(19) Europäisches Patentamt
European Patent Office
Office européen des brevets

(11) Publication number: **0 500 086 A1**

(12) **EUROPEAN PATENT APPLICATION**

(21) Application number: **92102813.0**

(51) Int. Cl.[5]: **C07D 209/16**, A61K 31/40

(22) Date of filing: **20.02.92**

(30) Priority: **22.02.91 GB 9103770**

(43) Date of publication of application:
**26.08.92 Bulletin 92/35**

(84) Designated Contracting States:
**AT BE CH DE DK ES FR GB GR IT LI LU MC NL PT SE**

(71) Applicant: **GLAXO GROUP LIMITED**
**Glaxo House, Berkeley Avenue**
**Greenford, Middlesex UB6 0NN(GB)**

(72) Inventor: **Bays, David Edmund**
**Glaxo Group Research Limited, Park Road**
**Ware, Hertfordshire SG12 0DG(GB)**

Inventor: **Bradshaw, John**
**Glaxo Group Research Limited, Park Road**
**Ware, Hertfordshire SG12 0DG(GB)**
Inventor: **Feniuk, Wasyl**
**Glaxo Group Research Limited, Park Road**
**Ware, Hertfordshire SG12 0DG(GB)**
Inventor: **North,Peter Charles**
**Glaxo Group Research Limited, Park Road**
**Ware, Hertfordshire SG12 0DG(GB)**

(74) Representative: **Filler, Wendy Anne**
**Glaxo Holdings p.l.c., Glaxo House, Berkeley Avenue**
**Greenford, Middlesex UB6 0NN(GB)**

(54) **Indole derivatives, their use as medicines and pharmaceutical compositions containing them.**

(57) The invention relates to indoles of general formula (I):

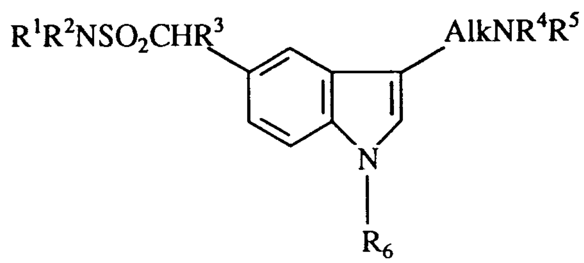

(I)

wherein $R^1$ represents a hydrogen atom or a $C_{1-6}$ alkyl or $C_{3-6}$ alkenyl group;
$R^2$ represents a hydrogen atom, a $C_{1-3}$ alkyl or $C_{3-6}$ alkenyl group, an aryl or ar($C_{1-4}$)alkylene group, or a $C_{5-7}$ cycloalkyl group;
$R^3$ represents a hydrogen atom or a $C_{1-3}$ alkyl group;
$R^4$ and $R^5$, which may be the same of different, each represents a hydrogen atom or a $C_{1-3}$alkyl or 2-propenyl group, or $R^4$ and $R^5$ together form an aralkylidine group;
$R^6$ represents a $C_{1-6}$ alkyl or $C_{3-6}$alkenyl group and
Alk represents an alkylene chain containing two or three carbons atoms which may be unsubstituted or substituted by not more than two $C_{1-3}$ alkyl groups, and physiologically acceptable salts and solvates (e.g. hydrates) thereof.

Compounds of general formula (I) are useful in treating conditions associated with cephalic pain, in particular migraine.

EP 0 500 086 A1

This invention relates to indole derivatives, to processes for their preparation, to pharmaceutical compositions containing them and to their medical use, in particular to compounds and compositions of use in the treatment of migraine.

It has been suggested that the pain of migraine may be associated with excessive dilatation of the cranial vasculature and known treatments for migraine include the administration of compounds having vasoconstrictor properties such as ergotamine. However, ergotamine is a non-selective vasoconstrictor which constricts blood vessels throughout the body and has undesirable and potentially dangerous side effects. Migraine may also be treated by administering an analgesic usually in combination with an antiemetic but such treatments are of limited value.

More recently, indole derivatives which are selective $5HT_1$-like receptors agonists and which exhibit selective vasoconstrictor activity have been described in the art as useful in the treatment of migraine. In our UK Patent No. 2124210B we describe indoles of the general formula

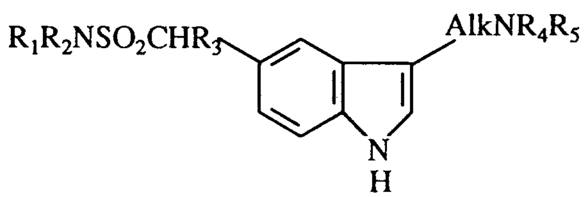

wherein $R_1$ represents a hydrogen atom or a $C_{1-6}$ alkyl or $C_{3-6}$ alkenyl group; $R_2$ represents a hydrogen atom or a $C_{1-3}$ alkyl, $C_{3-6}$ alkenyl, aryl, ar($C_{1-4}$)alkyl or $C_{5-7}$ cycloalkyl group; $R_3$ represents a hydrogen atom or a $C_{1-3}$ alkyl group; $R_4$ and $R_5$, which may be the same or different, each represents a hydrogen atom or a $C_{1-3}$ alkyl or propenyl group or $R_4$ and $R_5$ together form an aralkylidine group; and Alk represents an alkylene chain containing two or three carbon atoms which may be unsubstituted or substituted by not more than two $C_{1-3}$alkyl groups, and physiologically acceptable salts and solvates thereof.

As indicated in UK Patent No. 2124210B, compounds of the above formula selectively constrict the carotid arterial bed of the anaesthetised dog and are thus potentially useful for the treatment of migraine.

Preferred compounds described in UK Patent 2124210B include 3-[2-(methylamino)ethyl]-N-methyl-1H-indole-5-methanesulphonamide; 3-(2-aminoethyl)-N,N-dimethyl-1H-indole-5-methanesulphonamide; and 3-(2-aminoethyl)-N-(2-propenyl)-1H-indole-5-methanesulphonamide; and their physiologically acceptable salts and solvates, and a particularly preferred compound described in that specification is 3-(2-aminoethyl)-N-methyl-1H-indole-5-methanesulphonamide, and its physiologically acceptable salts and solvates.

In our UK Patent No. 2162522B we describe a particular compound which falls within the scope of the group of compounds claimed in UK Patent No. 2124210B, but which is not specifically disclosed therein, namely 3-[2-(dimethylamino)ethyl]-N-methyl-1H-indole-5-methanesulphonamide, and its physiologically acceptable salts and solvates. This compound possesses a combination of highly advantageous properties for the treatment of migraine and in this respect has advantages over compounds specifically disclosed in UK Patent 2124210B. Tests in anaesthetised dogs have shown that it potently and selectively constricts the carotid arterial bed following intravenous administration, and also that it is effectively and consistently well absorbed from the gastro-intestinal tract following intraduodenal administration. Its potent and selective vasoconstrictor action has also been demonstrated in vitro.

Certain 1-alkyl derivatives of the above indoles exhibit advantageous pharmacological properties, particularly improved bioavailability and/or increased duration of action.

Thus, the present invention provides an indole of general formula (I):

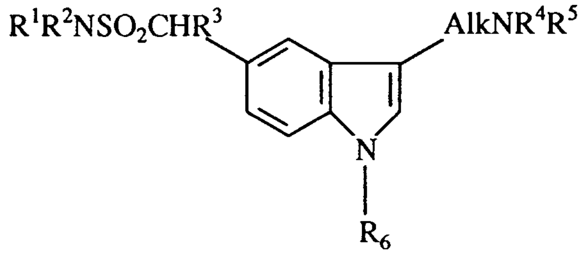

(I)

wherein $R^1$ represents a hydrogen atom or a $C_{1-6}$ alkyl or $C_{3-6}$ alkenyl group;
$R^2$ represents a hydrogen atom, a $C_{1-3}$ alkyl or $C_{3-6}$ alkenyl group, an aryl or ar($C_{1-4}$)alkylene group, or a $C_{5-7}$ cycloalkyl group;
$R^3$ represents a hydrogen atom or a $C_{1-3}$ alkyl group;
$R^4$ and $R^5$, which may be the same of different, each represents a hydrogen atom or a $C_{1-3}$alkyl or 2-propenyl group, or $R^4$ and $R^5$ together form an aralkylidine group;
$R^6$ represents a $C_{1-6}$ alkyl or $C_{3-6}$alkenyl group and
Alk represents an alkylene chain containing two or three carbons atoms which may be unsubstituted or substituted by not more than two $C_{1-3}$ alkyl groups, and physiologically acceptable salts and solvates (e.g. hydrates) thereof.

Referring to the general formula (I), an alkyl group may be a straight or branched chain alkyl group preferably containing from 1 to 3 carbon atoms such as a methyl, ethyl, propyl or isopropyl group. An alkenyl group preferably contains 3 or 4 carbon atoms and may be, for example, a propenyl, 2-propenyl or butenyl group. It will be appreciated that when $R^1$ or $R^2$ represents a $C_{3-6}$ alkenyl group, the double bond will not be adjacent to the nitrogen atom.

A cycloalkyl group in compounds of formula (I) preferably contains from 5 to 7 carbon atoms and may be, for example, a cyclopentyl, cyclohexyl or cycloheptyl group. An aryl group, either as such or as part of an ar($C_{1-4}$)alkylene or aralkylidene group, is preferably phenyl. The alkyl moiety in an ar($C_{1-4}$)alkylene group preferably contains 1 or 2 carbon atoms. An aralkylidene group is preferably an arylmethylidene group and may be, for example, a benzylidene group.

A preferred class of compounds represented by formula (I) is that in which $R^1$ represents a hydrogen atom or a $C_{1-6}$alkyl group and $R^2$ represents a hydrogen atom or a $C_{1-3}$ alkyl, $C_{3-6}$ alkenyl or ar($C_{1-4}$)-alkyl group.

Another preferred class of compounds of formula (I) is that in which $R^3$ represents a hydrogen atom.

A further preferred class of compounds of formula (I) is that in which $R^4$ and $R^5$, which may be the same or different, each represents a hydrogen atom or a $C_{1-3}$ alkyl group, for example a methyl or ethyl group. It is preferred that the total number of carbon atoms in $R^4$ and $R^5$ does not exceed two.

Another preferred class of compounds of formula (I) is that in which $R^6$ represents a methyl or an ethyl group.

A further preferred class of compounds of formula (I) is that in which Alk represents an ethylene group.

A further preferred class of compounds falling within the scope of formula (I) is that wherein $R^1$ represents a methyl group, $R^2$ and $R^3$ both represent a hydrogen atom, $R^4$ and $R^5$ both represent a methyl group and $R^6$ represents a methyl or an ethyl group.

Preferred compounds according to the invention include:
3-[2-(dimethylamino)ethyl]-N-methyl-1-ethyl-1H-indole-5-methanesulphonamide;
3-[2-(dimethylamino)ethyl]-N-methyl-1-methyl-1H-indole-5-methanesulphonamide;
and physiologically acceptable salts and solvates (for example, hydrates) thereof.

Suitable physiologically acceptable salts of the indoles of general formula (I) include acid addition slats formed with organic or inorganic acids for example hydrochlorides, hydrobromides, sulphates, fumarates, maleates and succinates. Other salts may be useful in the preparation of the compounds of general formula (I) e.g. creatinine sulphate adducts and oxalates.

Compounds of the invention are useful in treating conditions associated with cephalic pain such as cluster headache, chronic paroxysmal hemicrania, headache associated with vascular disorders, headache associated with substances or their withdrawal (for example drug withdrawal), tension headache and in particular migraine, and in alleviating the symptoms associated therewith. The successful treatment of such conditions is attributable to the potent and selective constriction of the carotid vasculature after administration in vivo.

Accordingly, the invention also provides a pharmaceutical composition which comprises at least one compound of formula (I) or a pharmaceutically acceptable salt or solvate (e.g. hydrate) thereof and formulated for administration by any convenient route. Such compositions are preferably in a form adapted for use in medicine, in particular human medicine, and can conveniently be formulated in conventional manner using one or more pharmaceutically acceptable carriers or excipients.

In a further aspect there is provided a compound of formula (I) or a salt or solvate thereof for use in therapy, in particular in human medicine. It will be appreciated that use in therapy embraces but is not necessarily limited to use of a compound of formula (I) or a salt or solvate thereof as an active therapeutic substance.

There is also provided as a further aspect of the invention the use of a compound of formula (I) in the preparation of a medicament for use in the treatment of conditions associated with cephalic pain such as

cluster headache, chronic paroxysmal hemicrania, headache associated with vascular disorders, headache associated with substances or their withdrawal (for example drug withdrawal), tension headache and in particular migraine.

In an alternative or further aspect there is provided a method for the treatment of a mammal, including man, comprising administration of an effective amount of a compound of formula (I) or salt or solvate thereof in particular in the treatment of conditions associated with cephalic pain and in alleviating the symptoms associated with cephalic pain and in alleviating the symptoms associated therewith.

It will be appreciated that reference to treatment is intended to include prophylaxis as well as the alleviation of established symptoms. Compounds according to the invention may be administered as the raw chemical but the active ingredient is preferably presented as a pharmaceutical formulation.

The active ingredient may conveniently be presented in unit dose form. A convenient unit dose formulation contains the active ingredient compound in an amount of from 0.1mg to 500mg.

The compounds according to the invention may for example be formulated for oral, sub-lingual, buccal, parenteral, rectal or intranasal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, methyl cellulose or hydrogenated edible fats); emulsifying agents (e.g. lechithin or acacia); non-aqueous vehicles (e.g. almond oil, oily esters or ethyl alcohol); and preservatives (e.g. methyl or propyl-p-hydroxybenzoates or sorbic acid).

Tablets for sub-lingual administration may be formulated in a similar manner to those for oral administration.

For buccal administration the compositions may take the form of tablets or lozenges formulated in conventional manner.

The compounds of the invention may be formulated for parenteral administration by injection, conveniently intravenous or subcutaneous injection, for example by bolus injection or continuous intravenous infusion. Formulations for injection may be presented in unit dosage form e.g. in ampoules or in multi-dose containers, with an added preservative.

The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glyceride.

For intranasal administration the compounds of the invention may be used, for example, as a liquid spray or powder or in the form of drops.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromthane, dichlorotetrafluoroethane, carbon dioxide or other suitable gas. In the case of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

It will be appreciated that the precise dose administered will depend on the age and condition of the patient, the particular compound used and the frequency and route of administration. The compound may be administered in single or divided doses and may be administered one or more times, for example 1 to 4 times per day.

A proposed dose of the compounds of the invention for oral, sub-lingual, parenteral, buccal, rectal or intranasal administration to man (of approximately 70kg bodyweight) for the treatment of migraine is 0.1 to 500mg of the active ingredient per unit dose which could be administered, for example, 1 to 4 times per

day.

For oral administration a unit dose will preferably contain from 2 to 500mg for example 100mg of the active ingredient. A unit dose for parenteral administration will preferably contain 0.2 to 25mg of the active ingredient.

Aerosol formulations are preferably arranged so that each metered dose or 'puff' delivered from a pressurised aerosol contains 0.2mg to 2mg of a compound of the invention, and capsules and cartridges delivered from an insufflator or an inhaler, contain 0.2mg to 20mg of a compound of the invention. The overall daily dose by inhalation with an aerosol will be within the range 1mg to 250mg. Administration may be several times daily, for example from 2 to 8 times, giving for example 1, 2 or 3 doses each time.

Dosages of the compounds of the invention for rectal and sub-lingual are similar to those for oral administration. For intranasal administration a unit dose will preferably contain from 10 to 100mg of the active ingredient.

The compounds of the invention may, if desired, be administered in combination with one or more other therapeutic agents, such as analgesics, anti-inflammatory agents and anti-nauseants, and formulated for administration by any convenient route in conventional manner. Appropriate doses will be readily appreciated by those skilled in the art.

Compounds of formula (I) may be prepared by the methods outlined below and which form a further aspect of the invention. In the following processes $R_1$, $R_2$, $R_3$, $R_4$, $R_5$, $R_6$ and Alk are as defined for formula (I) unless otherwise specified.

According to one general process (A), a compound of formula (I) may be prepared by cyclisation of a compound of formula (II)

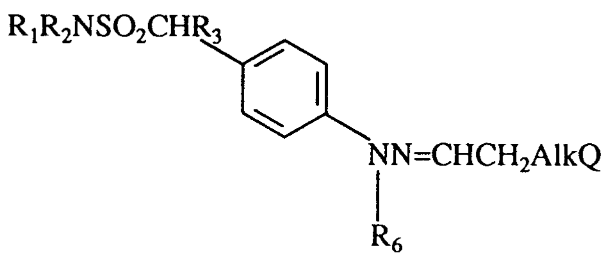

(II)

(wherein Q is the group $NR_4R_5$ or a protected derivative thereof or a leaving group such as a halogen atom (e.g. chlorine or bromine), or an acyloxy group such as acetoxy, chloroacetoxy, dichloroacetoxy, trifluoroacetoxy or p-nitrobenzoyloxy or a sulphonate group such as p-toluene sulphonate or methyl sulphonate).

Particularly convenient embodiments of the process are described below.

When Q is the group $NR_4R_5$ (or a protected derivative thereof), the process is desirably carried out in a suitable reaction medium, such as an aqueous organic solvent, for example, an aqueous alcohol (e.g. methanol, ethanol and isopropanol) or aqueous ether (e.g. dioxan) in the presence of an acid catalyst. (In some cases the acid catalyst may also act as the reaction solvent). Suitable acid catalysts include inorganic acids such as sulphuric or hydrochloric acid or organic carboxylic acids such as acetic acid. Alternatively the cyclisation may be carried out using polyphophate ester in a chlorinated solvent (e.g. chloroform) or using a Lewis acid such as zinc chloride in ethanol or boron trifluoride in acetic acid. The reaction may conveniently be carried out at temperatures of from 20 to 200$^0$C, preferably 50 to 125$^0$C.

When Q is a leaving group, such as a chlorine or bromine atom, the reaction may be effected in an aqueous organic solvent, such as an aqueous alcohol (e.g. methanol, ethanol or isopropanol) or an aqueous ether (e.g. dioxan), in the absence of an inorganic acid, conveniently at a temperature of from 20 to 200$^0$C, preferably 50 to 125$^0$C. This process results in the formation of a compound of formula (I) wherein $R_4$ and $R_5$ are both hydrogen atoms.

According to a particular embodiment of this process, compounds of general formula (I) may be prepared directly by the reaction of a compound of general formula (III):

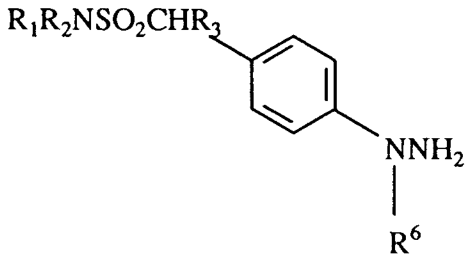

(III)

or a salt (e.g. the hydrochloride salt) thereof, with a compound of formula (IV):

$HCOCH_2AlkQ$ (IV)

(wherein Q is as defined above)
or a salt or protected derivative thereof (such as an acetal, for example, a dialkyl or cyclic acetal e.g. formed with an appropriate alkyl orthoformate or diol or protected as a bisulphite addition complex), using the appropriate conditions as described above for the cyclisation of a compound of general formula (II) (The Fischer-Indole Synthesis, B. Robinsion p 488 - Wiley 1982).

Compounds of general formula (II) may, if desired, be isolated as intermediates by reacting a compound of formula (III), or a salt or protected derivative thereof with a compound of formula (IV) or a salt or protected derivative thereof, in a suitable solvent, such as an aqueous alcohol (e.g. methanol) or an aqueous ether (e.g. dioxan) and at a temperature of, for example, from 20 to 30°C. If an acetal of a compound of formula (IV) is used it may be necessary to carry out the reaction in the presence of an acid (for example, acetic or hydrochloric acid).

As illustrated in the following general processes (B) and (C), the aminoalkyl substituent $-AlkNR_4R_5$ may be introduced at the 3-position by a variety of conventional techniques which may, for example, involve modification of a substituent at the 3-position or direct introduction of the aminoalkyl substituent into the 3-position.

Thus a further general process (B) for preparing compounds of general formula (I) involves reacting a compound of general formula (V):

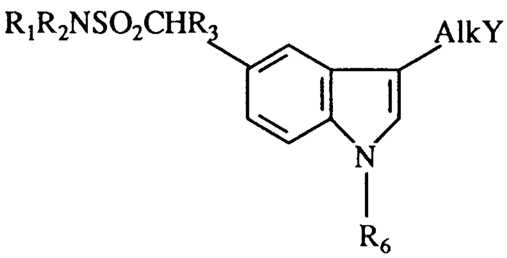

(V)

(wherein Y is a readily displaceable group)
or a protected derivative thereof, with a compound of formula $R_4R_5NH$.

This displacement reaction may conveniently be carried out on those compounds of formula (V) wherein the substituent group Y is a halogen atom (e.g. chlorine, bromine or iodine) or a group OR where OR is, for example, an acyloxy group, such as acetoxy, chloroacetoxy, dichloroacetoxy, trifluoroacetoxy or p-nitrobenzoyloxy or a sulphonate group (e.g. p-toluene sulphonate or methyl sulphonate).

The above reaction is conveniently effected in an inert organic solvent (optionally in the presence of water), examples of which include alcohols, e.g. ethanol; ethers, e.g. tetrahydrofuran; esters e.g. ethyl acetate; amides e.g. N,N-dimethylformamide; and ketones e.g. acetone. The process may be carried out at a temperature of, for example, -10 to +150°C, preferably 20 to 50°C.

The compounds of formula (V) wherein Y is a halogen atom may be prepared by reacting a hydrazine of formula (III) with an aldehyde (or a protected derivative thereof) of formula (IV) in which Q is a halogen atom, in an aqueous alcohol (e.g. methanol) or an aqueous ether (e.g. dioxan) containing an acid (e.g. acetic or hydrochloric acid) or by reacting a compound of general formula (V) wherein Y is a hydroxy group with the appropriate phosphorus trihalide. The intermediate alcohol, where Y is a hydroxy group, may also be used to prepare compounds of formula (V), wherein Y is a group OR, by acylation or sulphonylation with the

appropriate activated species (e.g. an anhydride or sulphonyl chloride) using conventional techniques.

Compounds of general formula (I) may also be prepared by another general process (C) involving reduction of a compound of general formula (VI):

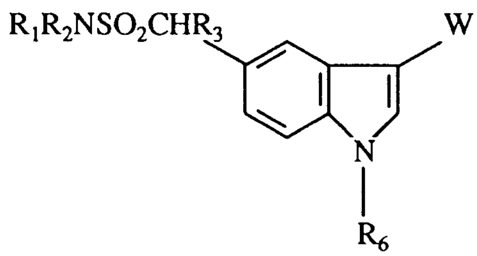

(VI)

(wherein W is a group capable of being reduced to give the required $AlkNR_4R_5$ group or a protected derivative thereof)

or a salt or protected derivative thereof)

The required Alk and $NR_4R_5$ groups may be formed by reduction steps which take place separately or together in any appropriate manner.

Examples of groups represented by the substituent group W include the following:-

$TNO_2$ (where T is Alk or an alkenyl group corresponding to the group (Alk)); $AlkN_3$; $AlkNR_4COR_9$; $-COCONR_4R_5$; $(CHR_7)CHR_8CN$; $CHR_8COZ$; $(CHR_7)_xCR_8=NOH$; $CH(OH)CHR_8NR_4R_5$; $COCHR_8Z$ (where $R_7$ and $R_8$ which may be the same or different, each represents a hydrogen atom or a $C_{1-3}$ alkyl group, Z is an azido group $N_3$ or the group $NR_4R_5$ or a protected derivative thereof, x is zero or 1 and $R_9$ is part of the group $R_5$ or the group $OR_c$ where $R_c$ is an alkyl or an aralkyl group).

Groups which may be reduced to the group Alk include corresponding unsaturated groups and corresponding groups containing one or more hydroxyl groups or carbonyl functions.

Groups which may be reduced to the group $NR_4R_5$ wherein $R_4$ and $R_5$ are both hydrogen include nitro, azido, hydroxyimino and nitrile groups. Reduction of a nitrile group yields the group $CH_2NH_2$ and thus provides a methylene group of the group Alk.

The required $NR_4R_5$ group wherein $R_4$ and/or $R_5$ are other than hydrogen may be prepared by reduction of a nitrile $(CHR_7)_xCHR_8CN$ or an aldehyde $(CHR_7)_xCHR_8CHO$ (where $R_7$, $R_8$ and x are as previously defined) in the presence of an amine, $R_4R_5NH$.

A particularly suitable method for preparing a compound of formula (I) wherein $R_4$ and/or $R_5$ is other than hydrogen, is reductive alkylation of the corresponding compound wherein $R_4$ and/or $R_5$ represents hydrogen, with an appropriate aldehyde or a ketone (e.g. acetaldehyde or benzaldehyde or acetone) in the presence of a suitable reducing agent. In some instances (e.g. for the introduction of the group $R_5$ where $R_5$ is ethyl) the aldehyde (e.g. acetaldehyde) may be condensed with the primary amine and the intermediate thus formed may subsequently be reduced using a suitable reducing agent.

A compound of general formula (I) wherein $R_5$ is a hydrogen atom, may also be prepared by reduction of a corresponding compound of general formula (I) wherein $R_5$ is a benzyl group, for example with hydrogen in the presence of a catalyst e.g. 10% palladium on carbon.

The required $NR_4R_5$ group wherein $R_4$ and/or $R_5$ are other than hydrogen may also be prepared by reduction of a corresponding amide, for example, $AlkNR_4COR_9$ (where $R_9$ is as previously defined).

It will be appreciated that the choice of reducing agent and reaction conditions will be dependent on the nature of the group W.

Suitable reducing agents which may be used in the above process for the reduction of compounds of formula (VI) wherein W represents, for example, the groups $TNO_2$, $AlkN_3$, $(CHR_7)_xCHR_8CN$, $(CHR_7)_xCR_8$-NOH, $CH(OH)CHR_8NR_4R_5$ (where T, $R_5$ and $R_7$ and $R_8$ and x are as previously defined) include hydrogen in the presence of a metal catalyst, for example Raney Nickel or a noble metal catalyst such as platinum, platinum oxide, palladium or rhodium, which may be supported, for example, on charcoal, kieselguhr or alumina. In the case of Raney Nickel hydrazine may also be used as the source of hydrogen. This process may conveniently be carried out in a solvent such as an alcohol e.g. ethanol, an ether, e.g. dioxan or tetrahydrofuran, an amide, e.g. dimethylformamide or an ester e.g. ethyl acetate, and at a temperature of from -10 to +50°C, preferably -5 to +30°C.

The reduction process may also be effected on compounds of formula (VI) wherein W represents, for example, the groups $TNO_2$, $AlkN_3$, $CH(OH)CHR_8NR_4R_5$ or $COCHR_8Z$ (where T, $R_8$ and Z are as previously defined), using an alkali metal or alkaline earth metal borohydride or cyanoborohydride e.g. sodium or

calcium borohydride or cyanoborohydride which process may conveniently be carried out in an alcohol such as propanol or ethanol and at a temperature of from 10 to 100°C, preferably 50 to 100°C. In some instances the reduction using a borohydride may be carried out in the presence of cobaltous chloride.

Reduction of compound of formula (VI) wherein W represents, for example, the group $TNO_2$, $AlkN_3$, $AlkNR_4COR_9$, $CHR_8COZ$, $(CHR_7)_xCR_8=NOH$, $CH(OH)CHR_8NR_4R_5$, $-CONR_4R_5$ and $COCHR_8Z$ (wherein T, $R_9$, $R_7$, $R_8$, Z and x are as previously defined) may also be carried out using a metal hydride such as lithium aluminium hydride. This process may be carried out in a solvent, for example, an ether such as tetrahydrofuran, and conveniently at a temperature of from -10 to +100°C, preferably 50 to 100°C.

A particular embodiment of this process includes the reduction of a compound of formula (VI) wherein W is the group $CHR_8CN$, for example, by catalytic reduction with hydrogen in the presence of a catalyst such as palladium or rhodium on alumina, optionally in the presence of an amine $HNR_4R_5$, or using lithium aluminium hydride.

The starting materials or intermediate compounds of general formula (VI) may be prepared by analogous methods to those described in U.K. Published Patent Application No. 2035310 and "A Chemistry of Heterocyclic Compounds - Indoles Part II" Chapter VI edited by W.J. Houlihan (1972) Wiley Interscience, New York.

A compound of formula (VI) wherein W is the group $AlkNHCOR_5$ may be prepared by acylation of the corresponding unsubstituted amine using conventional techniques.

The Fischer-indole cyclisation process may be employed to prepare a compound of formula (VI) wherein W is the group $(CHR_7)_xCHR_8CN$ or $CHR_7CHR_8NO_2$ in conventional manner.

According to a further general process (D) a compound of formula (I) may be prepared from a corresponding compound of formula (VII)

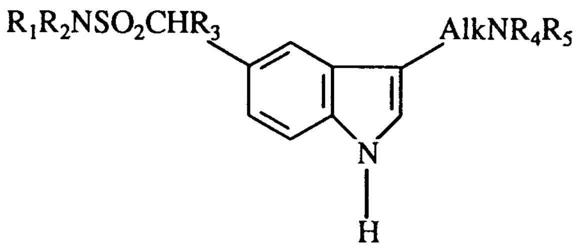

(VII)

with a suitable alkylating agent such as an alkyl halide (e.g. methyl or ethyl iodide), alkyl tosylate (e.g methyl tosylate) or dialkylsulphate (e.g. dimethylsulphate). The alkylation reaction is conveniently carried out in an inert organic solvent such as an amide (e.g. dimethylformamide), an ether (e.g. tetrahydrofuran) or an aromatic hydrocarbon (e.g. toluene) preferably in the presence of a base. Suitable bases include, for example, alkali metal hydrides, such as sodium hydride, alkali metal amides, such as sodium amide, alkali metal carbonates, such as sodium carbonate or alkali metal alkoxides such as sodium or potassium methoxide, ethoxide or t-butoxide.

The preparation of compounds of formula (VII) is described in UK Patent No. 2124210B.

The following reactions (E), in any appropriate sequence, may if necessary and/or desired be carried out subsequent to any of the above described processes:

(i) conversion of one compound of general formula (I) or a salt or protected derivative thereof into another compound of general formula (I);

(ii) removal of any protecting groups; and

(iii) conversion of a compound of general formula (I) or a salt thereof into a physiologically acceptable salt or solvate (e.g. hydrate) thereof.

Thus, a compound of formula (I) according to the invention may be converted into another compound of the invention using conventional procedures.

For example, a compound of general formula (I) wherein one or more of $R_1$, $R_2$, $R_4$ and $R_5$ are alkyl groups may be prepared from the corresponding compounds of formula (I) wherein one or more of $R_1$, $R_2$, $R_4$ and $R_5$ represent hydrogen atoms, by reaction with a suitable alkylating agent such as an alkyl halide (e.g. methyl or ethyl iodide), alkyl tosylate (e.g. methyl tosylate) or dialkylsulphate (e.g. dimethylsulphate). The alkylation reaction is conveniently carried out in an inert organic solvent such as an amide (e.g. dimethylformamide), an ether (e.g. tetrahydrofuran) or an aromatic hydrocarbon (e.g. toluene) preferably in the presence of a base. Suitable bases include, for example, alkali metal hydrides, such as sodium hydride, alkali metal amides, such as sodium amide, alkali metal carbonates, such as sodium carbonate or alkali

metal alkoxides such as sodium or potassium methoxide, ethoxide or t-butoxide.

It should be appreciated that in some of the above transformations it may be necessary or desirable to protect any sensitive groups in the molecule of the compound in question to avoid undesirable side reactions. For example, during any of the reaction sequences described above, it may be necessary to protect the group $NR_4R_5$, wherein $R_4$ and/or $R_5$ represents hydrogen, with a group easily removable at the end of the reaction sequence. Such groups may include, for example, aralkyl groups, such as benzyl, diphenylmethyl or triphenylmethyl; or acyl groups such as N-benzyloxycarbonyl or t-butoxycarbonyl or phthaloyl.

Subsequent cleavage of the protecting group may be achieved by conventional procedures. Thus an aralkyl group such as benzyl, may be cleaved by hydrogenolysis in the presence of a catalyst (e.g. palladium on charcoal) or sodium and liquid ammonia; an acyl group such as N-benzyloxycarbonyl may be removed by hydrolysis with, for example, hydrogen bromide in acetic acid or by reduction, for example by catalytic hydrogenation. The phthaloyl group may be removed by hydrazinolysis (e.g. by treatment with hydrazine hydrate) or by treatment with a primary amine (e.g. methylamine).

Where it is desired to isolate a compound of the invention as a physiologically acceptable salt, for example as an acid addition salt, this may be achieved by treating the free base of general formula (I), with an appropriate acid (e.g. succinic or hydrochloric acid) perferably with an equivalent amount in a suitable solvent (e.g. aqueous ethanol).

The starting materials or intermediate compounds for the preparation of the compounds according to this invention may be prepared by conventional methods analogous to those described in U.K. Published Patent Application No. 2035310.

As well as being employed as the last main step in the preparative sequence, the general methods indicated above for the preparation of the compounds of the invention may also be used for the introduction of the desired groups at an intermediate stage in the preparation of the required compound. Thus, for example, the required group at the 5-position may be introduced either before or after cyclisation to form the indole nucleus. It should therefore be appreciated that in such multi-stage processes, the sequence of reactions should be chosen in order that the reaction conditions do not affect groups present in the moleucle which are desired in the final products.

The invention is further illustrated by the following non-limiting examples. All temperatures are in $^{0}$C. Chromatography was performed on silica gel. DMF means dimethylformamide. DMSO means dimethylsulphoxide.

Example 1

3-[2-(Dimethylamino)ethyl]-N-methyl-1-ethyl-1H-indole-5-methanesulphonamide hemisuccinate

A suspension of 5% palladium on carbon (1.0g) in ethanol (15ml) was stirred under hydrogen for 45 min. A solution of 1-ethyl-3-(cyanomethyl)-N-methyl-1H-indole-5-methanesulphonamide (0.5g) in methanol (3ml) and 33% w/w dimethylamine in ethanol (30ml) were added to the catalyst slurry. The mixture was stirred under hydrogen at room temperature and atmospheric pressure for 65h. The catalyst was removed (filtration) and the filtrate was evaporated to dryness in vacuo to leave a pale brown gum (0.42g). This material was purified by flash chromatography eluting with dichloromethane:ethanol:ammonia (100:8:1 → 50:8:1), to give the free base.

A solution of the free base (0.09g) in ethanol (1ml) was treated with succinic acid (0.0164g) in ethanol (1ml). The resultant solution was evaporated to dryness and the residue stirred with anhydrous ether (2ml) for 72h. The suspension was filtered and the residue washed with anhydrous ether (5ml) to give the title compound as a hydroscopic cream powder m.p. 85-90$^{0}$.

T.l.c. $SiO_2$ dichloromethane:ethanol:0.88 ammonia (50:8:1) Rf 0.65.

Example 2

3-[2-(Dimethylamino)ethyl]-N-methyl-1-ethyl-1H-indole-5-methanesulphonamide hydrochloride

A stirred solution of 3-[2-(dimethylamino)ethyl]-N-methyl-1H-indole-5-methanesulphonamide (4.0g) in anhydrous DMF (150ml) was treated, under nitrogen at room temperature, with sodium hydride (1.08g of a 60% dispersion in oil) added portionwise while keeping the temperature below 25$^{0}$. The resultant solution was cooled and stirred at $^{-}12^{0}$ for 0.5h. A solution of diethylsulphate (0.88ml) in anhydrous DMF (50ml) was added over a period of 0.5h keeping the temperature between $^{-}9^{0}$ and $^{-}12^{0}$. The resultant solution was

allowed to attain room temperature with stirring over a period of 3.5h. 5N aqueous ammonia (50ml) was cautiously added over a period of 0.5h. The solvent was removed by rotary evaporation and the residual brown gum purified by flash chromatography eluting with dichloromethane:methanol:triethylamine (150:8:1 → 75:8:1) to give a yellow oil.

The oil (1.9g) was further purified by flash chromatography eluting with dichloromethane:ethanol:ammonia (100:8:1) to give a pale brown gum.

A solution of the gum (1.35g) in ethyl acetate (10ml) was added to ethanolic hydrogen chloride (20ml, containing 5.0mmol of HCl) with stirring. The solvent was evaporated and the residue triturated with ethyl acetate (10ml)/ethanol (5ml). The solid that crystallised out was filtered off and dried to give the title compound as a white powder m.p. 159-162$^0$.
T.l.c. $Et_3N$ deactivated $SiO_2$ ($CH_2Cl_2$:MeOH:$Et_3N$, 100:8:1) Rf 0.5.

| | | | | |
|---|---|---|---|---|
| Analysis Found: | C,53.4; | H,7.3; | N,11.6; | Cl,9.8. |
| $C_{16}H_{25}N_3O_2S$.HCl requires | C,53.6; | H,7.3; | N,11.7; | Cl,9.85%. |

## Example 3

### 3-[2-(Dimethylamino)ethyl]-N,1-dimethyl-1H-indole-5-methane sulphonamide

A stirred solution of 3-[2-(dimethylamino)ethyl]-N-methyl-1H-indole-5-methanesulphonamide (4.0g) in anhydrous DMF (150ml), under nitrogen, was treated with sodium hydride (1.08g of a 60% dispersion in oil. The mixture was cooled using a salt/ice bath and stirred for 20min. A solution of dimethylsulphate (0.64ml) in anhydrous DMF (850ml) was then added over a period of ca. 0.5h keeping the temperature between $^-2^0$ and $^-13^0$. After stirring for 1h (temp =ca 10$^0$) the solution was cooled to $^-5^0$ and a further portion of dimethylsulphate (0.51ml) in DMF (20ml) added over a period of 15min. Stirring was continued for 1h. Aqueous 5N ammonia (20ml) was added and the resulting solution concentrated under vacuum. The residue was diluted with water (200ml) and extracted with dichloromethane (4 x 100ml). The organic extracts were washed with aqueous 5N ammonia (2 x 100ml), dried and evaporated to give a pale brown oil. Flash chromatography using dichloromethane:ethanol:ammonia (150:8:1 → 100:8:1) as eluant gave the title compound as a white powder m.p. 89-93$^0$.
Water Assay Found: 0.74% $H_2O$ w/w.

| | | | |
|---|---|---|---|
| Analysis Found : | C,57.7; | H,7.6; | N,13.3. |
| $C_{15}H_{23}N_3O_2S.0.13H_2O$ requires | C,57.8; | H,7.5; | N,13.5%. |

## Example 4

### 3-[2-(Dimethylamino)ethyl]-N,N,1-trimethyl-1H-indole-5-methane sulphonamide

The title compound was isolated as a second product in the preparation of the compound of Example 3 and triturated with anhydrous ether to give the title compound as an off-white powder m.p. 115-116$^0$.
T.l.c. $SiO_2$ dichloromethane:ethanol:triethylamine (100:8:1) Rf 0.4.

## Example 5

### 3-[2-(Dimethylamino)ethyl]-N-methyl-1-(2-propenyl)-1H-indole-5-methanesulphonamide, hemisuccinate

A solution of 3-[2-(dimethylamino)ethyl]-N-methyl-1H-indole-5-sulphonamide (1.0g) in anhydrous DMF was cooled (salt/ice bath) and treated, under nitrogen, with sodium hydride (0.27g of a 60% dispersion in oil). The resulting suspension was stirred for 15min at $^-4^0$ and a solution of allyl chloride (0.27ml), in DMF (20ml) was added over a period of 20min (bath temperature kept at 0 → $^-5^0$). The solution was allowed to reach room temperature and stirred for 20h. 1N hydrochloric acid (10ml) was added and the mixture washed with ethyl acetate (20ml) and then basified with sodium bicarbonate solution. The basic solution was extracted with dichloromethane (3 x 75ml), dried and evaporated to give a liquid which was purified by flash

chromatography eluting with dichloromethane:ethanol:0.88 ammonia (100:8:1 → 50:8:1) to give the free base as an oil.

A solution of the free base (0.19g) in ethanol (0.5ml) was treated with a solution of succinic acid (0.034g) in ethanol (0.5ml). The solution was evaporated to dryness to give the title compound as a cream foam.

δ ($d_6$-DMSO), 2.34 (6H,s), 2.36 (2H,s), 2.58 (3H,d), 2.6-2.92 (4H,2xm), 4.38 (2H,s), 4.78 (2H,d), 4.99-5.2 (2H,2xdq), 5.98 (1H,m), 6.86 (1H,q), 7.14 (1H,dd), 7.21 (1H,s), 7.39 (1H,d), 7.56 (1H,d).

Example 6

3-[2-(Dimethylamino)ethyl]-N-methyl-1-pentyl-1H-indole-5-methane sulphonamide oxalate

A solution of 3-[2-(dimethylamino)ethyl]-N-methyl-1H-indole-5-methanesulphonamide (1.0g) in anhydrous DMF (40ml) was cooled (salt/ice bath) and treated, under nitrogen, with sodium hydride (0.27g of a 60% dispersion in oil). The resulting suspension was stirred for 15min at $^{-}11^{0}$ (cooling bath temperature). A solution of 1-bromopentane (0.42ml) in anhydrous DMF (20ml) was added over a period of 20min and the mixture allowed to warm to room temperature. Stirring was continued at room temperature for 72h. The reaction mixture was cautiously treated with water (10ml), 2N hydrochloric acid (5ml) and water (200ml) and then extracted with cyclohexane (150ml) and dichloromethane (3 x 100ml). Evaporation of the dried dichloromethane extracts gave a viscous oil (0.58g). Flash chromatography eluting with dichloromethane:ethanol:ammonia (100:8:1) gave the free base as an oil.

The free base (0.13g) in ethanol (0.5ml) was treated with a solution of oxalic acid (0.03g) in ethanol (0.5ml). The white crystalline product was filtered off and washed with ethanol and then ether to give the title compound as a white powder m.p. 172-178$^{0}$.

δ ($d_6$-DMSO), 0.83 (3H,t), 1.14-1.36 (4H,m), 1.74 (2H,m), 2.42 (3H,s), 2.81 (6H,s), 3.0-3.3 (4H,m), 4.14 (2H,t), 4.47 (1H,brs), 4.58 (2H,s), 7.16 (1H,dd), 7.31 (1H,s), 7.48 (1H,d), 7.69 (1H,d).

Example 7

3-[2-(Dimethylamino)ethyl]-N-methyl-N,1-dipentyl-1H-indole-5-methane sulphonamide oxalate

The free base of the title compound was isolated as a by-product in the preparation of the compound of Example 6.

The free base (0.25g) in ethanol (1.0ml) was treated with a solution of oxalic acid (50.8mg) in ethanol (0.5ml). The crystalline product was filtered off, washed with ethanol and then ether to give the title compound as a white powder (0.21g) m.p. 132-137$^{0}$.

δ ($d_6$-DMSO), 0.84 (6H,2xt), 1.1-1.49 (10H,m), 1.76 (2H,m), 2.71 (3H,s), 2.78 (6H,s), 2.95 (2H,t), 3.0-3.27 (4H,m), 4.14 (2H,t), 4.43 (2H,s), 7.19 (1H,dd), 7.3 (1H,s), 7.47 (1H,d), 7.61 (1H,d).

The following example illustrates a pharmaceutical formulation according to the invention. The active ingredient is an indole of general formula (I) or a physiologically acceptable salt or solvate thereof.

| Tablets for oral administration | |
|---|---|
| | mg/tablet |
| Active ingredient | 100 |
| Magnesium Stearate | 1.00 |
| Anhydrous lactose | 99 |

The active ingredient is sieved and blended with the anhydrous lactose and magnesium stearate. The mix is then compressed into tablets using a Manesty F3 tablet machine fitted with 8.0mm concave punches.

**Claims**

**1.** A compound of general formula (I):

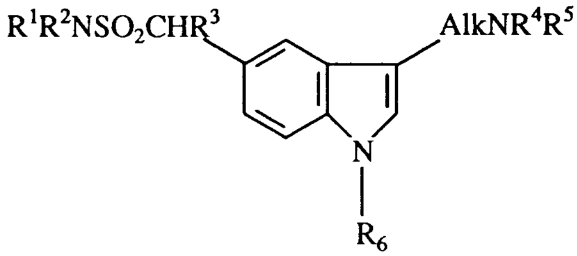

(I)

wherein $R^1$ represents a hydrogen atom or a $C_{1-6}$ alkyl or $C_{3-6}$ alkenyl group;
$R^2$ represents a hydrogen atom, a $C_{1-3}$ alkyl or $C_{3-6}$ alkenyl group, an aryl or ar($C_{1-4}$)alkylene group, or a $C_{5-7}$ cycloalkyl group;
$R^3$ represents a hydrogen atom or a $C_{1-3}$ alkyl group;
$R^4$ and $R^5$, which may be the same of different, each represents a hydrogen atom or a $C_{1-3}$alkyl or 2-propenyl group, or $R^4$ and $R^5$ together form an aralkylidine group;
$R^6$ represents a $C_{1-6}$ alkyl or $C_{3-6}$alkenyl group and
Alk represents an alkylene chain containing two or three carbons atoms which may be unsubstituted or substituted by not more than two $C_{1-3}$ alkyl groups, and physiologically acceptable salts and solvates thereof.

**2.** Compounds as claimed in Claim 1 in which $R^1$ represents a hydrogen atom or a $C_{1-6}$alkyl group and $R^2$ represents a hydrogen atom or a $C_{1-3}$alkyl, $C_{3-6}$alkenyl or ar($C_{1-4}$)alkyl group.

**3.** Compounds as claimed in Claim 1 or Claim 2 in which $R^3$ represents a hydrogen atom.

**4.** Compounds as claimed in any one of Claims 1 to 3 in which $R^4$ and $R^5$, which may be the same or different, each represents a hydrogen atom or a $C_{1-3}$alkyl group.

**5.** Compounds as claimed in any one of Claims 1 to 4 in which $R^6$ represents a methyl or an ethyl group.

**6.** Compounds as claimed in anyone of Claims 1 to 5 in which Alk represents an ethylene group.

**7.** Compounds as claimed in any one of Claims 1 to 6 in which $R^1$ represents a methyl group, $R^2$ and $R^3$ both represent a hydrogen atom, $R^4$ and $R^5$ both represent a methyl group and $R^6$ represents a methyl or an ethyl group.

**8.** A pharmaceutical composition which comprises a compound of formula (I) as claimed in any one of Claims 1 to 7 or a pharmaceutically acceptable salt or solvate thereof together with one or more pharmaceutically acceptable carriers or excipients.

**9.** A compound of formula (I) as claimed in any one of Claims 1 to 7 for use in therapy.

**10.** Use of a compound of formula (I) as claimed in any one of Claims 1 to 7 in the preparation of a medicament for use in the treatment of conditions associated with cephalic pain such as cluster headache, chronic paroxysmal hemicrania, headache associated with vascular disorders, headache associated with substances or their withdrawal (for example drug withdrawal), tension headache and in particular migraine.

European Patent Office

# EUROPEAN SEARCH REPORT

Application Number

EP 92 10 2813

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A,D | GB-A-2 162 522 (GLAXO GROUP LIMITED (UNITED KINGDOM))<br>* claim 1; example 15(ii)*<br>--- | 1-10 | C07D209/16<br>A61K31/40 |
| A<br><br>D | DE-A-3 320 521 (GLAXO GROUP LTD.)<br>* claims 1-12,15; examples *<br>& GB-A-2 124 210<br>--- | 1-10 | |
| A | EP-A-0 242 939 (GLAXO GROUP LIMITED)<br>* claims 1,9; examples *<br>--- | 1-10 | |
| A | GB-A-2 081 717 (GLAXO GROUP LIMITED)<br>* page 1, line 35 - line 42; claims 1,11 *<br>----- | 1-10 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 06 MAY 1992 | P. BOSMA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)